(19) Europäisches Patentamt
European Patent Office
Office européen des brevets

(11) **EP 3 632 334 A1**

(12) **EUROPEAN PATENT APPLICATION**

(43) Date of publication:
**08.04.2020 Bulletin 2020/15**

(51) Int Cl.:
**A61B 8/08** *(2006.01)*　　　　**A61M 25/01** *(2006.01)*
**H01L 41/047** *(2006.01)*　　**H01L 41/313** *(2013.01)*
**A61B 17/34** *(2006.01)*

(21) Application number: **18198820.5**

(22) Date of filing: **05.10.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Koninklijke Philips N.V.**
**5656 AG Eindhoven (NL)**
• **B. Braun Melsungen AG**
**34212 Melsungen (DE)**

(72) Inventors:
• **STAPERT, Hendrik Roelof**
**5656 AE Eindhoven (NL)**

• **LIJTEN, Gerardus Franciscus Cornelis Maria**
**5656 AE Eindhoven (NL)**
• **DE WIJS, Willem-Jan Arend**
**5656 AE Eindhoven (NL)**
• **SHIBAYAMA, Yuichi**
**34212 Melsungen (DE)**
• **THORENZ, Mathias**
**34212 Melsungen (DE)**

(74) Representative: **de Haan, Poul Erik et al**
**Philips International B.V.**
**Philips Intellectual Property & Standards**
**High Tech Campus 5**
**5656 AE Eindhoven (NL)**

(54) **INTERVENTIONAL DEVICE WITH AN ULTRASOUND TRANSDUCER**

(57)　　An interventional device 100, 200, 300 includes an elongate shaft 101 having a longitudinal axis A - A', an ultrasound transducer 102, an adhesive layer 103, and a protective tube 104 formed from a heat-shrink material. The ultrasound transducer 102 is disposed on the elongate shaft 101 such that the ultrasound transducer 102 has an axial extent L along the longitudinal axis A - A'. At least along the axial extent L of the ultrasound transducer 102 the protective tube 104 surrounds the ultrasound transducer 102 and the adhesive layer 103 is disposed between the ultrasound transducer 102 and the protective tube 104.

FIG. 1A

EP 3 632 334 A1

**Description**

FIELD OF THE INVENTION

The invention relates to an interventional device with an ultrasound transducer. The interventional device may be used in various interventional procedures in the medical field. In one contemplated application the ultrasound transducer is an ultrasound detector used to track a position of the interventional device respective an ultrasound field of an ultrasound imaging probe.

BACKGROUND OF THE INVENTION

Interventional procedures in the medical field increasingly use ultrasound to gain more information about, or to treat, a patient's anatomy. In this regard, ultrasound devices may be equipped with an ultrasound transducer, defined herein as an ultrasound detector, or an ultrasound emitter, or a device that is capable of both detecting and emitting ultrasound signals, for use in sensing and actuation applications such as tracking, imaging, or treatment.

In one exemplary application described in more detail in document "A Non-disruptive Technology for Robust 3D Tool Tracking for Ultrasound-Guided Interventions" by Jay Mung, Francois Vignon, and Ameet Jain, in MICCAI 2011, Part I, LNCS 6891, pp. 153-160, 2011, A. Martel, and T. Peters (Eds.), an ultrasound detector is attached to a medical needle and used to track the needle position respective the ultrasound field of a beamforming ultrasound imaging probe based on the timing of ultrasound signals detected by the detector.

Another method for attaching an ultrasound transducer to an interventional device for use in a tracking application is disclosed in document WO2016207041A1. This document describes transfer stack for transferring a portion of a foil within a perimeter that includes a transducer to an article such as a medical device or a medical needle. The transfer stack includes a carrier substrate, a foil having a transducer incorporated therein, and the transducer is laterally surrounded by a perimeter. The foil is separable from the carrier substrate by overcoming a first peel retaining force. An adhesive layer is also attached to the foil. The adhesive layer is configured to provide adhesion between the foil and an article such that when the article is attached to the foil via the adhesive layer the foil is separable from the surface of the article by overcoming a second peel retaining force. The second peel retaining force (PRF2) is greater than the first peel retaining force.

Another document WO2017013224A1 is also relevant to attaching an ultrasound transducer to an interventional device for the purposes of ultrasound-based tracking. This document describes a transducer laminate in which electrical contact is made between electrical conductors and a transducer layer. The transducer laminate includes two adhesive-coated foils, whose adhesive coatings are arranged to face each other. At a first position along the length of the two electrical conductors the two electrical conductors are sandwiched between the adhesive coatings of the two adhesive-coated foils, and the transducer layer is also sandwiched between the two electrical conductors such that electrical contact is made with the electrodes on the transducer layer. At a second position along the length of the two electrical conductors the two electrical conductors are sandwiched between the adhesive coatings of the two adhesive-coated foils and there is no transducer layer sandwiched between the two electrical conductors.

Other exemplary applications such as intravascular ultrasound, i.e. IVUS, imaging, also include one or more ultrasound transducers on an interventional device such as a catheter, in order to generate images of the vasculature.

Despite recent progress in this field there remains room to improve the attachment of ultrasound transducers to interventional devices in such application areas.

SUMMARY OF THE INVENTION

The present invention seeks to improve the attachment of ultrasound transducers to interventional devices. Some known solutions to this problem may suffer from the ingress of moisture in the vicinity of the ultrasound transducer, which may affect transducer performance. Other known solutions to this problem may suffer from the interventional device having an irregular topology, particularly in the vicinity of the ultrasound transducer. As a result, a medical professional user may experience a variable resistance to insertion when inserting such interventional devices into the body.

In order to address one or more of the aforementioned drawbacks, an interventional device is provided. A related ultrasound-based position determination system that incorporates the interventional device, and a related method of manufacturing the interventional device are also provided. The interventional device includes an elongate shaft having a longitudinal axis, an ultrasound transducer, an adhesive layer, and a protective tube formed from a heat-shrink material. The ultrasound transducer is disposed on the elongate shaft such that the ultrasound transducer has an axial extent along the longitudinal axis. Moreover, at least along the axial extent of the ultrasound transducer the protective tube surrounds the ultrasound transducer and the adhesive layer is disposed between the ultrasound transducer and the protective tube.

The protective tube may reduce the ingress of moisture into the ultrasound transducer. The protective tube may also provide a smoother topology over the ultrasound transducer and thereby provide for a smoother introduction of the interventional device into the body. By forming the protective tube from a heat shrink material a reliable manufacturing method is provided. Moreover, the inventors have discovered that by disposing the adhesive layer between the ultrasound transducer and the protective tube, improved ultrasound transducer performance may be achieved. It has been found that when such a protective tube is disposed over the ultrasound transducer, typically a thin layer of air is trapped between the ultrasound transducer and the protective tube. This layer of air acts as an ultrasound reflector and/ or attenuator, and due to its irregular thickness rotationally about the protective tube, may consequently introduce a rotational variation in ultrasound sensitivity and/ or radiated ultrasound signal strength. The adhesive layer reduces the tendency for such a layer of air to form, thereby improving the ultrasound transducer's sensitivity and/ or radiated ultrasound signal strength, and the rotational variability in these parameters. Moreover, the adhesive layer also reduces the risk of moisture reaching the ultrasound transducer, which acts to maintain the ultrasound transducer's performance over time.

Further aspects are described with reference to the appended claims. Further advantages from the described invention will also be apparent to the skilled person.

BRIEF DESCRIPTION OF THE FIGURES

Fig. 1 illustrates orthogonal views of an interventional device 100 that includes an ultrasound transducer 102 and an adhesive layer 103.

Fig. 2 illustrates orthogonal views of an interventional device 200 that includes an ultrasound transducer 102 in the form of a band, and an adhesive layer 103.

Fig. 3 illustrates orthogonal views of an interventional device 300 that includes an ultrasound transducer 102 in the form of a band, an adhesive layer 103, and an electrical shield layer 105.

Fig. 4 illustrates orthogonal views of an interventional device 200 in which ultrasound transducer 102 is provided by a transducer strip 410 that is wrapped in the form of a spiral around elongate shaft 101 of interventional device 200.

Fig. 5 illustrates a transducer strip 410 that includes an ultrasound transducer 102 and an adhesive layer 103.

Fig. 6 illustrates various views of a transducer strip 410 that includes a first electrical conductor 115, a second electrical conductor 116, a first electrode 117 and a second electrode 118.

Fig. 7 illustrates an exemplary ultrasound-based position determination system 700 that includes an interventional device.

Fig. 8 illustrates in Fig. 8A a method of manufacturing interventional device 100, 200, 300 by rolling 842 elongate shaft 101 of the interventional device across an ultrasound transducer transfer stack 840, and in Fig. 8B a cross sectional end-view of the stack along D - D', and in Fig. 8C a cross sectional end-view of the stack along E - E'.

Fig. 9 illustrates sensitivity measurements in arbitrary units versus angular position in degrees for two experimental ultrasound transducers, N121-15 and N121-22, wrapped around an interventional device in the absence of any adhesive layer.

Fig. 10 illustrates sensitivity measurements in arbitrary units versus angular position in degrees for two experimental ultrasound transducers, N126-9 and N126-13, wrapped around an interventional device in the presence of an adhesive layer.

DETAILED DESCRIPTION OF THE INVENTION

In order to illustrate the principles of the present invention an interventional device in the form of a medical needle is described with particular reference to an exemplary position tracking application in which the positon of an ultrasound detector on the needle is determined respective the ultrasound field of a beamforming ultrasound imaging system. It is however to be appreciated that the invention may also be used in other application areas that employ ultrasound transducers such as ultrasound imaging and treatment applications. It is also to be appreciated that whilst reference is made to an ultrasound transducer in the form of an ultrasound detector, the ultrasound transducer may alternatively be an ultrasound emitter, or indeed be capable of both detecting and emitting ultrasound signals, or indeed comprise both an ultrasound emitter and an ultrasound detector. The invention also finds application with other interventional devices than a medical needle, including without limitation a catheter, a guidewire, a biopsy device, a guidewire, a pacemaker lead, an intravenous line or a surgical tool in general. The interventional device may be used in a wide variety or medical procedures, for example from routine needle insertion for regional anesthesia, to biopsies and percutaneous ablation of cancer, and to more advanced interventional procedures.

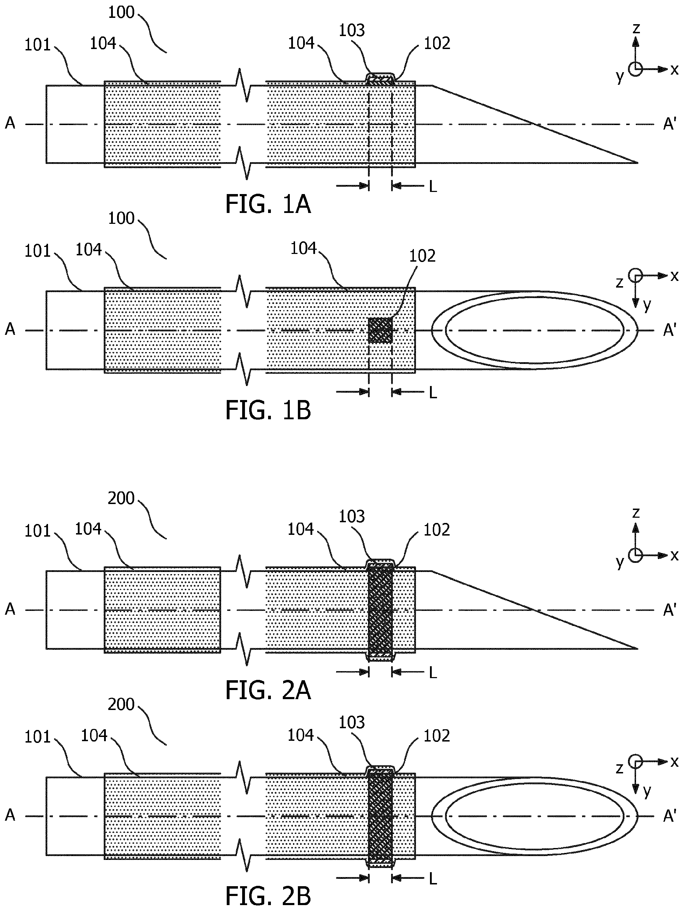

Fig. 1 illustrates orthogonal views of an interventional device 100 that includes an ultrasound transducer 102 and an adhesive layer 103. Interventional device 100 has an elongate shaft with longitudinal axis A - A'. Ultrasound transducer 102 is disposed on elongate shaft 101 such that ultrasound transducer 102 has an axial extent, L, along longitudinal axis A - A'. Moreover, at least along axial extent L of ultrasound transducer 102, protective tube 104 surrounds ultrasound transducer 102 and adhesive layer 103 is disposed between ultrasound transducer 102 and protective tube 104. Protective tube 104 is formed from a heat-shrink material.

In a preferred implementation, ultrasound transducer 102 is formed from a piezoelectric material. Various so-called hard or soft piezoelectric materials may be used. The piezoelectric material may for example be a polymer such as Polyvinylidene fluoride, i.e. PVDF, PVDF co-polymer such as polyvinylidene fluoride trifluoroethylene (P(VDF-TrFE)) layer, or PVDF ter-polymer such as P(VDF-TrFE-CTFE). One exemplary supplier of a suitable PVDF polymer is Goodfellow, Cambridge, UK. Alternatively, ultrasound transducer 102 may be a capacitive micromachined ultrasound transducer, i.e. a CMUT device. In a preferred example, ultrasound transducer 102 comprises a single transducer, although the use of an array of two or more such transducers is also contemplated.

Various adhesive materials are contemplated for use as adhesive layer 103 in Fig. 1. Adhesives such as epoxies may for example be used, including EPO-TEK 301, and the use of UV-curable adhesives is also contemplated. In a preferred implementation, adhesive layer 103 is provided by a pressure sensitive adhesive, i.e. PSA. Pressure sensitive adhesives are a class of materials that form an adhesive bond upon application of pressure. Advantageously, pressure sensitive adhesives provide a reliable bond upon contraction of heat-shrink protective tube 104 and thereby a robust structure that is quick to assemble. Suitable pressure sensitive adhesives include product 8211CL made by the 3M Corporation. These may be supplied as PSA-coated polymer sheets, i.e. foils, such as product 9019 supplied by the 3M Corporation. Foils with PSA on one or both surfaces are available. PSA-coated polymer sheets are typically provided with a removable release layer that is peeled away to reveal the adhesive coating and thereby protect the adhesive layer until its adhesive properties are required. The foils may be formed from a range of polymer materials, for example Polyethylene terephthalate, PET, Polyimides, PI, or Polyamides, PA. Typically the foils are formed from an electrically insulating material.

Various heat-shrink materials are contemplated for use as protective tube 104. Polyolefins and fluropolymers including PVDF, HDPE, LDPE, EMA are amongst the materials that are contemplated. Suitable materials for protective tube 104 include polyester, PET, materials provided by Nordson Medical, Colorado, USA and product MT5500 supplied by the Raychem Corporation, USA.

In the exemplary interventional device 100 illustrated in Fig. 1, elongate shaft 101 is provided by a medical needle. Ultrasound transducer 102 is disposed adjacent the needle bevel at the distal end of the needle. As described later, this position is selected in order to track the position of the tip of the needle, although this position and the medical needle itself are only exemplary. In another implementation, ultrasound transducer 102 may alternatively be closer to the proximal end of elongate shaft 101 than to the distal end, i.e. the end having the bevel.

In the exemplary interventional device 100 illustrated in Fig. 1, ultrasound transducer 102 is illustrated in the form of a patch having axial extent, L, along longitudinal axis A - A'. Other shapes of transducer are also contemplated. Moreover, at least along axial extent L of ultrasound transducer 102, protective tube 104 surrounds ultrasound transducer 102, and adhesive layer 103 is disposed between ultrasound transducer 102 and protective tube 104. In Fig. 1, a substantial portion of the length of elongate shaft 101 is illustrated as being covered by protective tube 104 in order to provide a smooth outer surface along the entire length of elongate shaft 101 but this is purely illustrative. It is however preferred that protective tube 104 extends axially in one or both directions beyond the axial extent L of ultrasound transducer 102 in order to provide good sealing against the ingress of moisture to ultrasound transducer 102. Moreover, adhesive layer 103 is illustrated as only being along the axial extent of ultrasound transducer 102. In other implementations adhesive layer 103 may extend in one or both directions axially beyond the axial extent L of the ultrasound transducer 102, which may be advantageous in relaxing alignment tolerances.

In one exemplary fabrication process, interventional device 100 in Fig. 1 may be made by first disposing ultrasound transducer 102 on elongate shaft 102. An adhesive may be used for this purpose, or attachment via Van der Waals forces may suffice. Adhesive layer 102 may subsequently be applied to the outer surface of ultrasound transducer 102 in the form of a liquid. Subsequently, protective tube 104 may be arranged on elongate shaft 101 over ultrasound transducer 102 and heated in order to radially contract protective tube 104. Subsequently the adhesive may be cured, for example by waiting for a predetermined time to elapse, or by heating, or by exposing the adhesive to UV radiation when UV-curable adhesive is used.

In an alternative exemplary fabrication process, a pressure sensitive adhesive may be used for adhesive layer 102. A layer of PSA may for example be deposited on the outer surface of ultrasound transducer 102 after its attachment to elongate shaft 102. Alternatively ultrasound transducer 102 may be attached to a portion of PSA-coated foil having PSA on both surfaces prior to its attachment to elongate shaft 101. Upon application of heat to protective tube 104, the inner surface of protective tube 104 becomes attached to the outermost PSA layer as the protective tube contracts.

Fig. 2 illustrates orthogonal views of an interventional device 200 that includes an ultrasound transducer 102 in the form of a band, and an adhesive layer 103. References to features in Fig. 2 have the same meaning as those described in relation to Fig. 1. In Fig. 2, ultrasound transducer 102 is disposed on the interventional device in the form of a band wrapped around the longitudinal axis A - A' of the elongate shaft. The band is illustrated as continuous and lying in a plane that is perpendicular to longitudinal axis A - A' but may alternatively have one or more gaps around its circumference. Moreover the band may alternatively be tilted with respect to a plane perpendicular to longitudinal axis A - A'. The fabrication processes described in relation to Fig. 1 may also be used to make the device of Fig. 2. In addition to the advantages of Fig. 1, ultrasound transducer 102 in Fig, 2 provides for ultrasound sensing and/ or emission around longitudinal axis A - A' of elongate shaft 101.

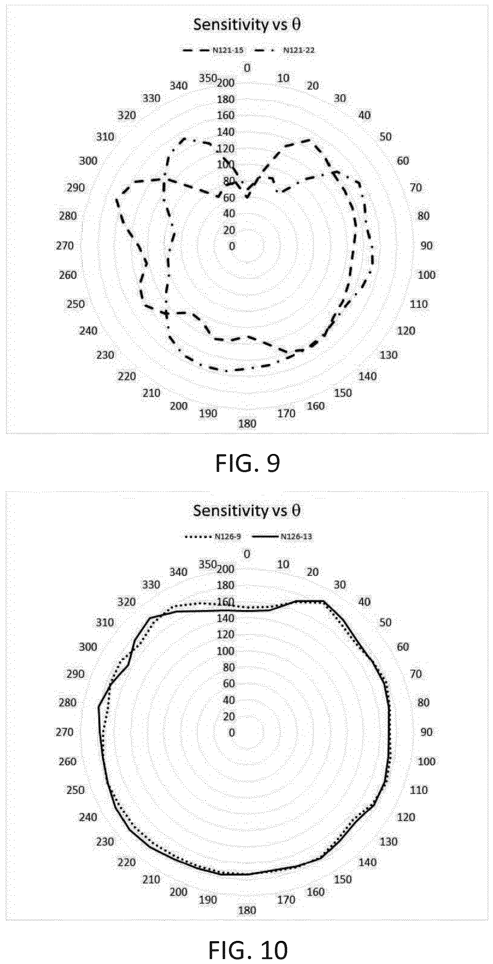

Fig. 9 and Fig. 10 illustrate the benefits of using adhesive layer 102 in interventional device 200 illustrated in Fig. 2. Thereto, Fig. 9 illustrates sensitivity measurements in arbitrary units versus angular position in degrees for two experimental ultrasound transducers, N121-15 and N121-22, wrapped around an interventional device in the absence of any adhesive layer. Fig. 10 illustrates sensitivity measurements in arbitrary units versus angular position in degrees for two experimental ultrasound transducers, N126-9 and N126-13, wrapped around an interventional device in the presence of an adhesive layer. The ultrasound transducer used in each sensitivity measurement illustrated in Fig. 9 and Fig. 10 was a PVDF foil that was wrapped around each of needles N121-15, N121-22, N126-9 and N126-13 in the form of a band, as illustrated in Fig. 2. As can be seen in Fig. 9, in the absence of an adhesive layer, large variations in sensitivity with rotational angle are observed for each of the needles N121-15, N121-22. A significant improvement in terms of both absolute sensitivity as well as reduced variation in sensitivity is illustrated in the results for needles N126-9 and N126-13 of Fig. 10, highlighting the benefit of adhesive layer 103.

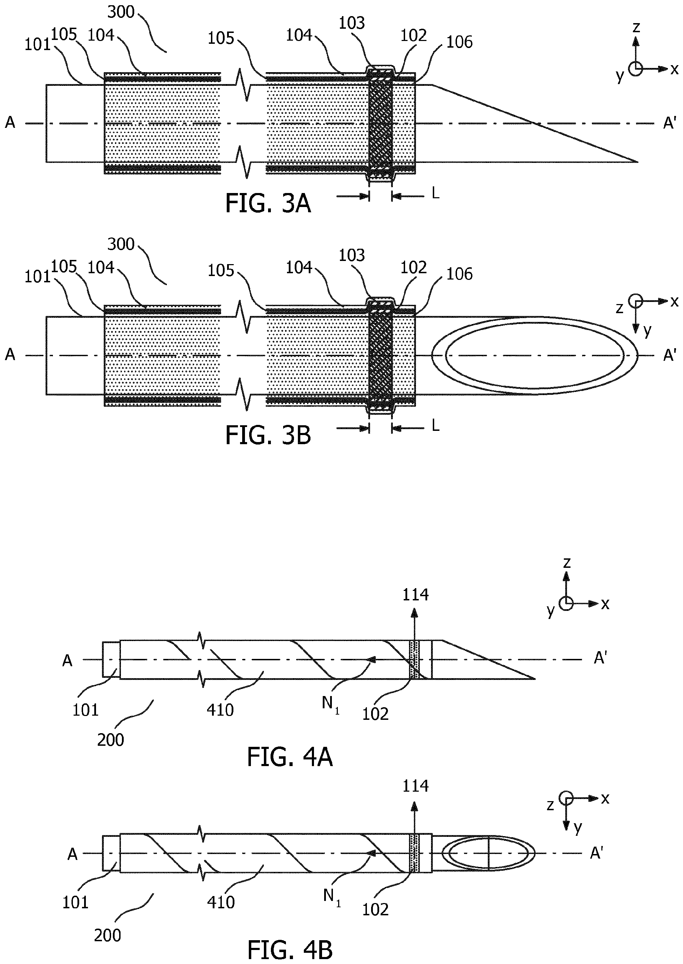

Fig. 3 illustrates orthogonal views of an interventional device 300 that includes an ultrasound transducer 102 in the form of a band, an adhesive layer 103, and an electrical shield layer 105. References to features in Fig. 2 have the same meaning as those described in relation to Fig. 2. In addition to the features in Fig. 2, Fig. 3 includes electrical shield layer 105 that is disposed between ultrasound transducer 102 and protective tube 104. Electrical shield layer 105 may be formed from a conductor such as copper or gold, and may alternatively be in the form of a mesh. Electrical shield layer 105 serves to electrically shield ultrasound transducer 102, and also any electrical conductors connected thereto (not shown in Fig. 3) that may be present, and thereby reduce electromagnetic interference. Electrical insulator layer 106 may also, as illustrated in Fig. 3 be disposed between ultrasound transducer 102 and shield layer 105. Electrical insulator layer 106 serves to electrically insulate ultrasound transducer 102 from shield layer 105. Insulator layer 106 may be formed from an insulator such as a polymer, for Polyethylene terephthalate (PET), Polyimide (PI), or Polyamide (PA). Electrical shield layer 105 and electrical insulator layer 106 may also be used in the same manner in interventional device 100 of Fig. 1.

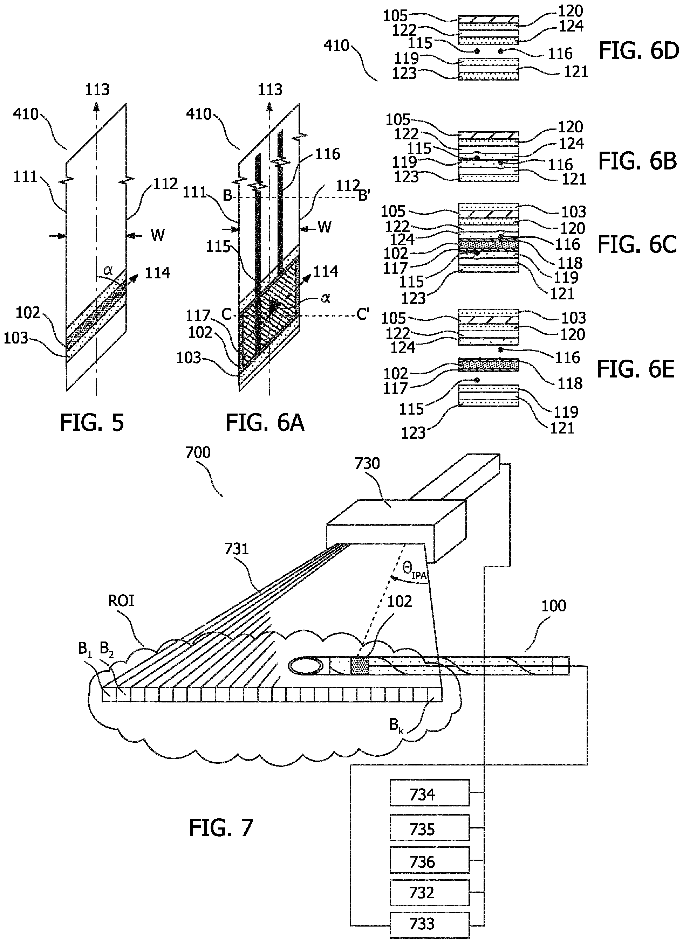

Another contemplated method of attaching ultrasound transducer 102 to interventional device is to provide ultrasound transducer 102 as a transducer strip and to wrap this in the form of a spiral around elongate shaft 101 of interventional device 200. Thereto, Fig. 4 illustrates orthogonal views of an interventional device 200 in which ultrasound transducer 102 is provided by a transducer strip 410 that is wrapped in the form of a spiral around elongate shaft 101 of interventional device 200. Adhesive layer 103 and protective tube 104 are omitted from Fig. 4 for ease of illustration but may be applied in the same manner as described in relation to Fig. 2. The use of such a transducer strip advantageously results in a smooth outer profile to the interventional device, particularly when electrical conductors in transducer strip 410, not shown in Fig. 4, are also wrapped in the form of a spiral around elongate shaft 101 of interventional device 200. Exemplary transducer strips 410 for use in this method are illustrated in Fig. 5 and Fig. 6.

Fig. 5 illustrates a transducer strip 410 that includes an ultrasound transducer 102 and an adhesive layer 103. The support for ultrasound transducer 102 and adhesive layer 103 illustrated as a rhomboidal shape may be provided by a polymer film such as Polyethylene terephthalate, PET, Polyimide, PI, or Polyamide, PA. A polymer film in the forms of a PSA-coated foil as described herein may for example provide this support. The arrangement of Fig. 4 may be obtained by wrapping exemplary transducer strip 410 of Fig. 4 around elongate shaft 101 in the form of a spiral as illustrated in Fig. 4.

With reference to Fig. 5, transducer strip 410 includes first edge 111 and opposing second edge 112, these edges being separated by a width dimension W. First edge 111 and second edge 112 each extend along length direction 113 of transducer strip 410. Length direction 113 is orthogonal to the direction in which width dimension W is measured. Transducer strip 410 includes ultrasound transducer 102 that extends along transducer direction 114. Transducer direction 114 forms an acute angle, $\alpha$, with respect to length direction 113 of transducer strip 410. Moreover, adhesive layer 103 covers ultrasound transducer 102. When the exemplary transducer strip 410 of Fig. 5 is wrapped in the form of a spiral around elongate shaft 101 of interventional device 200 in Fig. 4, ultrasound transducer 102 forms a band around elongate shaft 101.

Optionally, width dimension W in Fig. 5 may be defined such that adjacent first and second edges 111, 112 of consecutive turns of the spiral abut or overlap one another.

In order for consecutive turns of the spiral to abut, i.e. just touch, one another, the following equation should be satisfied:

$$W=\pi\cdot D\cdot Sin(\alpha) \qquad\qquad\qquad \text{Equation 1}$$

Wherein $\alpha$ is the acute angle defined by transducer direction 114 with respect to length direction 113, and D is the diameter of elongate shaft 101. By arranging that W exceeds the above value, consecutive turns of the spiral overlap one another. Likewise by arranging that W is less than this value a small gap may be provided between consecutive turns of the spiral.

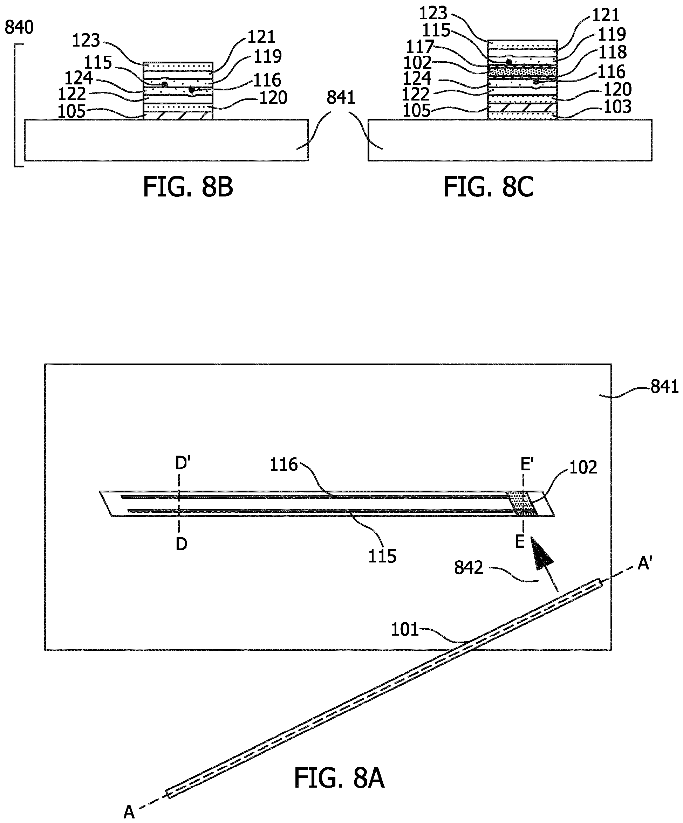

The spiral wrapping arrangement of Fig. 4 provides a simple method of attaching ultrasound transducer 102 to elongate shaft 101. The interventional device may for example be rolled across the transducer strip and attached to the interventional device by means of an adhesive as described later with reference to Fig. 8. The abutting or overlapping adjacent turns act to provide, respectively, a smooth outer surface to interventional device 110 and thereby lower resistance to insertion in a body, and avoid the exposure of material underlying the wrapped transducer strip.

Thus, together, Fig. 4 and Fig. 5 illustrate an interventional device 300 wherein ultrasound transducer 102 and adhesive layer 103 are provided by transducer strip 410. Transducer strip 410 includes ultrasound transducer 102, adhesive layer 103, first edge 111 and opposing second edge 112, first edge 111 and second edge 112 being separated by a width dimension W, and first edge 111 and second edge 112 each extending along length direction 113 of transducer strip 410. Ultrasound transducer 102 is disposed on transducer strip 410 and extends along transducer direction 114 that forms acute angle $\alpha$ with respect to length direction 113 of transducer strip 410. Adhesive layer 103 covers the ultrasound transducer 102. Transducer strip 410 is wrapped in the form of a spiral around elongate shaft 101 of interventional device 200 such that the ultrasound transducer 102 forms a band around elongate shaft 101.

Fig. 6 illustrates various views of a transducer strip 410 that includes a first electrical conductor 115, a second electrical conductor 116, a first electrode 117 and a second electrode 118. Fig. 6A illustrates a plan view, Fig. 6B illustrates a section along B - B', Fig. 6C illustrates a section along C - C', Fig. 6D illustrates an exploded section along B - B', and Fig. 6E illustrates an exploded section along C - C'. Transducer strip 410 of Fig. 6 is an alternative to that of Fig. 5, and may likewise be wrapped in the form of a spiral around elongate shaft 101 of interventional device 200. In addition to the items of Fig. 5, Fig. 6 includes first electrical conductor 115 and second electrical conductor 116. First electrical conductor 115 and second electrical conductor 116 each extend along length direction 113 of transducer strip 410. Moreover, ultrasound transducer 102 further includes first electrode 117 and second electrode 118. First electrical conductor 115 is in electrical contact with first electrode 117, and second electrical conductor 116 is in electrical contact with second electrode 118 such that when transducer strip 410 is wrapped in the form of a spiral around elongate shaft 101 of interventional device 200, as illustrated in Fig. 4, first electrical conductor 115 and second electrical conductor 116 each extend along elongate shaft 101 for making electrical contact with ultrasound transducer 102 at an axially-separated position along elongate shaft 101 with respect to ultrasound transducer 102.

As illustrated in the exploded views of Fig. 6B - Fig. 6E, transducer strip 410 may comprise various laminated foils. This provides a simple fabrication technique. Thereto, at first position, B - B', Fig. 6B and Fig. 6D include first foil strip 121 that comprises a layer of pressure sensitive adhesive 119, 123, PSA, on each surface; first electrical conductor 115; second electrical conductor 116; adhesive layer 103; second foil strip 122 comprising a layer of pressure sensitive adhesive 120, 124, PSA, on each surface; and electrical shield layer 105. First foil strip 121 and second foil strip 122 are arranged to sandwich first electrical conductor 115 and second electrical conductor 116, and electrical shield layer 105 is disposed on outwards-facing PSA layer 124 of second foil strip 122. At second position C - C', Fig. 6C and Fig. 6E further include ultrasound transducer 102 that has first electrode 117 and second electrode 118 on each of its surfaces. At second position C - C' ultrasound transducer 102 having first electrode 117 and second electrode 118 are sandwiched between PSA layer 119 of first foil strip 121 and PSA layer 124 of second foil strip 122 such that electrical contact is made between first electrical conductor 115 and first electrode 117, and between second electrical conductor 116 and second electrode 118.

As mentioned above, the interventional devices described herein may for example be used in an ultrasound-based tracking application. In this, the ultrasound transducer may detect, or emit, or both detect and emit, ultrasound signals, and the position of the ultrasound transducer may thus be determined based on ultrasound signals transmitted between ultrasound detector 102 and a beamforming ultrasound imaging system.

Thereto, Fig. 7 illustrates an exemplary ultrasound-based position determination system 700 that includes an interventional device. In Fig. 7, ultrasound-based position determination system 700 includes a beamforming ultrasound imaging probe 730 which is in communication with image reconstruction unit 732, imaging system processor 736, imaging system interface 735 and display 734. Together, units 730, 732, 734, 735 and 736 form a conventional ultrasound imaging system. The units 732, 734, 735 and 736 are conventionally located in a console that is in wired or wireless communication with beamforming ultrasound imaging probe 730. Some of units 732, 734, 735 and 736 may alternatively be incorporated within beamforming ultrasound imaging probe 730 as for example in the Philips Lumify ultrasound imaging system. Beamforming ultrasound imaging probe 730 generates ultrasound field 731. In Fig. 7, a 2D beamforming ultrasound imaging probe 730 is illustrated that includes a linear ultrasound transceiver array that transmits and receives ultrasound energy within an ultrasound field 731 which intercepts region of interest ROI. The ultrasound field is fan-shaped in Fig. 7 and includes multiple ultrasound beams $B_{1..k}$ that together provide the illustrated image plane. Note that whilst Fig. 7 illustrates a fan-shaped beam the invention is not limited to a particular shape of ultrasound field or indeed to a planar ultrasound field. Beamforming ultrasound imaging probe 730 may also include electronic driver and receiver circuitry, not shown, that is configured to amplify and/ or to adjust the phase of signals it transmits or receives in order to generate and detect ultrasound signals in ultrasound beams $B_{1..k}$.

In-use the above-described conventional ultrasound imaging system is operated in the following way. An operator may plan an ultrasound procedure via imaging system interface 735. Once an operating procedure is selected, imaging system interface 735 triggers imaging system processor 736 to execute application-specific programs that generate and interpret the signals transmitted to and detected by beamforming ultrasound imaging probe 730. A memory, not shown, may be used to store such programs. The memory may for example store ultrasound beam control software that is configured to control the sequence of ultrasound signals transmitted by and/or received by beamforming ultrasound imaging probe 730. The function of image reconstruction unit 732 may alternatively be carried out by imaging system processor 736. Image reconstruction unit 732 provides a reconstructed ultrasound image corresponding to ultrasound field 731 of beamforming ultrasound imaging probe 730. Image reconstruction unit 732 thus provides an image corresponding to the image plane defined by ultrasound field 731 and which intercepts region of interest ROI. The image is subsequently displayed on display 734. The reconstructed image may for example be an ultrasound Brightness-mode "B-mode" image, otherwise known as a "2D mode" image, a "C-mode" image or a Doppler mode image, or indeed any ultrasound image.

Also shown in Fig. 7 is interventional device 100, exemplified by a medical needle, which includes piezoelectric transducer 102. In this exemplary application of the interventional device, interventional device 102, or more specifically ultrasound transducer 102 disposed thereon, may be tracked respective ultrasound field 731 based on signals provided by position determination unit 733. Position determination unit is in communication with units 730, 732, 734, 735 and 736, i.e. the conventional ultrasound imaging system, as illustrated by the interconnecting links. Position determination unit 733 is also in communication with ultrasound transducer 102, which communication may for example be wired or wireless. The function of position determination unit 733 may in some implementations be carried out by a processor of the conventional ultrasound imaging system.

In-use, the position of ultrasound transducer 102 is computed respective ultrasound field 731 by position determination unit 733 based on ultrasound signals transmitted between beamforming ultrasound imaging probe 730 and ultrasound transducer 102.

In one configuration ultrasound transducer 102 is a detector that receives ultrasound signals corresponding to beams $B_{1..k}$. Position determination unit 733 identifies the position of ultrasound transducer 102 by comparing the ultrasound signals detected by piezoelectric transducer 102. Position determination unit 733 subsequently provides an icon in the reconstructed ultrasound image based on the computed position of ultrasound transducer 102. More specifically the comparison determines the best fit position of ultrasound transducer 102 respective ultrasound field 731 based on i) the amplitudes of the ultrasound signals corresponding to each beam $B_{1..k}$ that are detected by ultrasound transducer 102, and based on ii) the time delay, i.e. time of flight, between emission of each beam $B_{1..k}$ and its detection by ultrasound transducer 102. This may be illustrated as follows. When ultrasound transducer 102 is in the vicinity of ultrasound field 731, ultrasound signals from the nearest of beams $B_{1..k}$ to the transducer will be detected with a relatively larger amplitude whereas more distant beams will be detected with relatively smaller amplitudes. Typically the beam that is detected with the largest amplitude is identified as the one that is closest to ultrasound transducer 102. This beam defines the in-plane angle $\theta_{IPA}$ between beamforming ultrasound imaging probe 730 and ultrasound transducer 102. The corresponding range depends upon the time delay, i.e. the time of flight, between the emission of the largest-amplitude beam $B_{1..k}$ and its subsequent detection. The range may be determined by multiplying the time delay by the speed of ultrasound propagation. Thus, the range and corresponding in-plane angle $\theta_{IPA}$ of the beam detected with the largest amplitude may be used to identify the best-fit position of ultrasound transducer 102 respective ultrasound field 731.

In the above example, ultrasound beams $B_{1..k}$ are imaging beams. In another configuration ultrasound beams $B_{1..k}$ may be dedicated tracking beams that are emitted in tracking frames between consecutive imaging frames in predetermined directions by beamforming ultrasound imaging probe 730.

In yet another configuration ultrasound transducer 102 may be an emitter that emits one or more ultrasound pulses. Such pulses may for example be emitted during tracking frames that are interleaved between consecutive imaging frames of the conventional ultrasound imaging system. In such a tracking frame, beamforming ultrasound imaging probe 730 may operate in a receive-only mode in which it listens for ultrasound signals originating from the vicinity of ultrasound field 731. Beamforming ultrasound imaging probe 730 is thus configured as a one-way receive-only beamformer during such tracking frames. Position determination unit 733 identifies from which beam of virtual beams $B_{1..k}$ the pulse(s) originated by applying delays to the receiver elements of beamforming ultrasound imaging probe 730. The delays correspond to each of virtual beams $B_{1..k}$. As in the ultrasound detector configuration above, position determination unit 733 may use a comparison procedure that, based on the maximum amplitude and time of flight, identifies the closest beam $B_{1..k}$ to the position at which the ultrasound signal was emitted. Position determination unit 733 subsequently provides an icon in the reconstructed ultrasound image based on the identified position of ultrasound transducer 102.

In another configuration ultrasound transducer 102 may be configured to act as both a receiver and an emitter. In this configuration ultrasound transducer 102 may be triggered to emit one or more ultrasound pulses upon receipt of an ultrasound signal from beamforming ultrasound imaging probe 730. In this way the pulse(s) emitted by ultrasound transducer 102 during an imaging mode are received by beamforming ultrasound imaging probe 730 appear as an echo in the reconstructed ultrasound at an in-plane angular position, i.e. in an image line, that corresponds to the relevant beam $B_{1..k}$. Ultrasound transducer 102 thus appears as a bright spot in the reconstructed image. Position determination unit 733 may subsequently identify this bright spot in the reconstructed image and thus compute a position of ultrasound transducer 102 respective ultrasound field 731.

In the above-described ultrasound-based position determination system 730 the dependence of the sensitivity profile, or emission profile, of piezoelectric transducer 102, or more specifically its magnitude and/ or dependence on rotational angle of the interventional device, may impact its positioning respective ultrasound field 731. Thereto, the use of the above-described interventional device has the benefits of improved reliability and sensitivity.

It is to be appreciated that the exemplified beamforming ultrasound imaging probe 730 is only one example of a beamforming ultrasound imaging system in which interventional device 100 may be used. Interventional device 100 also finds application in ultrasound-based position determination systems that include other types of 2D or 3D beamforming ultrasound imaging systems. These may include for example a "TRUS" transrectal ultrasonography probe, an "IVUS" intravascular ultrasound probe, a "TEE" transesophageal probe, a "TTE" transthoracic probe, a "TNE" transnasal probe, an "ICE" intracardiac probe. Moreover, it is to be appreciated that the interventional device 100 also finds application in other sensing and actuation applications in the medical field beyond position tracking.

Fig. 8 illustrates in Fig. 8A a method of manufacturing interventional device 100, 200, 300 by rolling 842 elongate shaft 101 of the interventional device across an ultrasound transducer transfer stack 840, and in Fig. 8B a cross sectional end-view of the stack along D - D', and in Fig. 8C a cross sectional end-view of the stack along E - E'. The items in Fig. 8 correspond to the items with the same references in Fig. 6. Fig. 8 additionally includes a substrate 801 which may for example be formed from glass or plastic, and which serves as a layer on which the stack may be constructed. Moreover, in Fig. 8 transducer transfer stack 840 is illustrated in reverse order to that in Fig. 6 since Fig. 8 illustrates the stack prior to its transfer to elongate shaft 101; i.e. with adhesive layer 103 adjacent to substrate 841.

Thereto, with reference to Fig. 8 a method of manufacturing an interventional device 100, 200, 300 includes the steps of:

providing an ultrasound transducer transfer stack 840 that includes:

a substrate 841
first foil strip 121 comprising a layer of pressure sensitive adhesive 119, 123, i.e. PSA, on each surface
ultrasound transducer 102
adhesive layer 103
second foil strip 122 comprising a layer of pressure sensitive adhesive 120, 124, i.e. PSA, on each surface; and
electrical shield layer 105.

Electrical shield layer 105, the first foil strip 121, the ultrasound transducer 102 and second foil strip 122 are arranged layerwise on substrate 841 such that at first position D - D' along transducer transfer stack 840, electrical shield layer 105 is disposed between substrate 841 and second foil strip 122 and first foil strip 121 is arranged on second foil strip 122 with one of the PSA layers 123 of first foil strip 121 facing outwards with respect to substrate 841, and such that at second position E - E' along transducer transfer stack 840, adhesive layer 103 is disposed between substrate 841 and electrical shield layer 105 and second foil strip 122 is arranged on electrical shield layer 105 and ultrasound transducer 102 is arranged on second foil strip 122 and first foil strip 121 is arranged on ultrasound transducer 102 with one of the PSA layers 123 of first foil strip 121 facing outwards with respect to substrate 841.

The method also includes rolling 842 elongate shaft 101 of interventional device 100, 200, 300 across outwards-facing PSA layer 123 of first foil strip 121 such that outwards-facing PSA layer of first foil strip 123 adheres to elongate shaft 101 and such that first foil strip 121 and the ultrasound transducer 102 and the adhesive layer 103 and the second foil strip 122 and the electrical shield layer 105 become attached to the elongate shaft 101 with adhesive layer 103 facing outwards with respect to the elongate shaft 101.

The method also includes arranging a protective tube 104 comprising a heat-shrink material over a portion of the elongate shaft 101 to cover at least the ultrasound transducer 102, and applying heat to the protective tube 104 such that the protective tube 104 contracts radially with respect to the longitudinal axis A - A' of elongate shaft 101 and such that adhesive layer 103 adheres to an inner surface of the protective tube 104.

Whilst not illustrated in Fig. 8 there may additionally be a release layer disposed between substrate 841 and ultrasound transducer transfer stack 840. This may help to release adhesive layer 103 from substrate 841. Alternatively the relative strengths of PSA layers 123 and adhesive layer 103 may be specified to ensure that layer 103 is released during rolling 842. Moreover, as illustrated, prior to rolling step 42, strips 121, 122 are arranged at an angle to longitudinal axis of elongate shaft 101 such that the foil is wrapped in the form of a spiral around the elongate shaft. The first foil strip 121 and the second foil strip 122 are, as illustrated, preferably in the form of an elongate strip having a length direction and a width direction, the length being greater than the width. Adhesive layer 103 may be provided as descried above in relation to Fig. 1. Preferably adhesive layer 103 comprises a pressure sensitive adhesive, PSA, layer. Moreover, as illustrated in Fig. 8B and 8C, ultrasound transducer transfer stack 840 may further comprise at both the first position D - D' and the second position E - E' a first electrical conductor 115 and a second electrical conductor 116 disposed between the first foil strip 121 and the second foil strip 122; the first electrical conductor 115 and the second electrical conductor 116 being in electrical contact with the ultrasound transducer 102.

In summary, an interventional device has been provided. The interventional device 100, 200, 300 includes an elongate shaft 101 having a longitudinal axis A - A', an ultrasound transducer 102, an adhesive layer 103, and a protective tube 104 formed from a heat-shrink material. The ultrasound transducer 102 is disposed on the elongate shaft 101 such that the ultrasound transducer 102 has an axial extent L along the longitudinal axis A - A'. At least along the axial extent L of the ultrasound transducer 102 the protective tube 104 surrounds the ultrasound transducer 102 and the adhesive layer 103 is disposed between the ultrasound transducer 102 and the protective tube 104.

Various embodiments and options have been described in relation to the interventional device, and it is noted that the various embodiments may be combined to achieve further advantageous effects. Any reference signs in the claims should not be construed as limiting the scope of the invention.

## Claims

1. An interventional device (100, 200, 300) comprising:

    an elongate shaft (101) having a longitudinal axis (A - A');
    an ultrasound transducer (102),
    an adhesive layer (103); and
    a protective tube (104) formed from a heat-shrink material;
    wherein the ultrasound transducer (102) is disposed on the elongate shaft (101) such that the ultrasound transducer (102) has an axial extent (L) along the longitudinal axis (A - A');
    wherein at least along the axial extent (L) of the ultrasound transducer (102) the protective tube (104) surrounds the ultrasound transducer (102) and the adhesive layer (103) is disposed between the ultrasound transducer (102) and the protective tube (104).

2. The interventional device (100, 200, 300) according to claim 1 wherein the adhesive layer (103) comprises a pressure sensitive adhesive, PSA, layer.

3. The interventional device (100, 200, 300) according to claim 1 wherein the adhesive layer (103) extends axially beyond the axial extent (L) of the ultrasound transducer (102).

4. The interventional device (200, 300) according to claim 1 wherein the ultrasound transducer (102) is disposed on the interventional device in the form of a band wrapped around the longitudinal axis (A - A') of the elongate shaft.

5. The interventional device (300) according to claim 1 further comprising an electrical shield layer (105);
    wherein the electrical shield layer (105) is disposed between the ultrasound transducer (102) and the protective tube (104).

6. The interventional device (100, 200, 300) according to claim 1 wherein the ultrasound transducer (102) comprises a capacitive micromachined ultrasound transducer, CMUT, or a piezoelectric material.

7. The interventional device (100, 200, 300) according to claim 1 wherein the ultrasound transducer (102) comprises a piezoelectric polymer layer selected from the group:

    Polyvinylidene fluoride, PVDF, PVDF co-polymer such as polyvinylidene fluoride trifluoroethylene (P(VDF-TrFE)) layer, or PVDF ter-polymer such as P(VDF-TrFE-CTFE).

8. The interventional device (100, 200, 300) according to claim 1 wherein the elongate shaft (101) is provided by a needle, a catheter, a guidewire, a biopsy device, a pacemaker lead, an intravenous line, or a surgical tool.

9. The interventional device (100, 200, 300) according to claim 1 wherein the elongate shaft (101) is provided by a needle having a proximal end, a distal end, and a bevel;
   wherein the bevel is disposed at the distal end of the needle; and
   wherein the ultrasound transducer (102) is disposed closer to the distal end than to the proximal end.

10. The interventional device (200, 300) according to claim 1 wherein the ultrasound transducer (102) and the adhesive layer (103) are provided by a transducer strip (410);
    wherein the transducer strip (410) comprises:

    the ultrasound transducer (102);
    the adhesive layer (103);
    a first edge (111) and an opposing second edge (112), the first edge (111) and the second edge (112) being separated by a width dimension (W), and the first edge (111) and the second edge (112) each extending along a length direction (113) of the transducer strip (410); and

    wherein the ultrasound transducer (102) is disposed on the transducer strip (410) and extends along a transducer direction (114) that forms an acute angle (α) with respect to the length direction (113) of the transducer strip (410);
    wherein the adhesive layer (103) covers the ultrasound transducer (102);
    wherein the transducer strip (410) is wrapped in the form of a spiral around the elongate shaft (101) of the interventional device (200, 300) such that the ultrasound transducer (102) forms a band around the elongate shaft (101).

11. The interventional device (200, 300) according to claim 10 wherein the transducer strip (410) further comprises a first electrical conductor (115) and a second electrical conductor (116), the first electrical conductor (115) and the second electrical conductor (116) extending along the length direction (113) of the transducer strip (410), and wherein the ultrasound transducer (102) further comprises a first electrode (117) and a second electrode (118);
    wherein the first electrical conductor (115) is in electrical contact with the first electrode (117) and the second electrical conductor (116) is in electrical contact with the second electrode (118) such that when the transducer strip (410) is wrapped in the form of a spiral around the elongate shaft (101) of the interventional device (200, 300) the first electrical conductor (115) and the second electrical conductor (116) each extend along the elongate shaft (101) for making electrical contact with the ultrasound transducer (102) at an axially-separated position along the elongate shaft (101) with respect to the ultrasound transducer (102).

12. Ultrasound-based position determination system (700) comprising:

    an interventional device (100, 200, 300) according to claim 1;
    a beamforming ultrasound imaging probe (730) configured to generate an ultrasound field (731);
    an image reconstruction unit (732) configured to provide a reconstructed ultrasound image corresponding to the ultrasound field (731) of the beamforming ultrasound imaging probe (730);
    a position determination unit (733) configured to compute a position of the ultrasound transducer (102) of the interventional device (100, 200, 300) respective the ultrasound field (731) based on ultrasound signals transmitted between the beamforming ultrasound imaging probe (730) and the ultrasound transducer (102), and to provide an icon in the reconstructed ultrasound image based on the computed position of the ultrasound transducer (102).

13. Method of manufacturing the interventional device according to claim 1, the method comprising the steps of:

    providing an ultrasound transducer transfer stack (840), the ultrasound transducer transfer stack comprising:

    a substrate (841);
    a first foil strip (121) comprising a layer of pressure sensitive adhesive (119, 123), PSA, on each surface;
    an ultrasound transducer (102);
    an adhesive layer (103);
    a second foil strip (122) comprising a layer of pressure sensitive adhesive (120, 124), PSA, on each surface; and
    an electrical shield layer (105);

wherein the electrical shield layer (105), the first foil strip (121), the ultrasound transducer (102) and the second foil strip (122) are arranged layerwise on the substrate (841) such that at a first position (D - D') the electrical shield layer (105) is disposed between the substrate (841) and the second foil strip (122) and the first foil strip (121) is arranged on the second foil strip (122) with one of the PSA layers (123) of the first foil strip (121) facing outwards with respect to the substrate (841), and such that at a second position (E - E') the adhesive layer (103) is disposed between the substrate (841) and the electrical shield layer (105) and the second foil strip (122) is arranged on the electrical shield layer (105) and the ultrasound transducer (102) is arranged on the second foil strip (122) and the first foil strip (121) is arranged on the ultrasound transducer (102) with one of the PSA layers (123) of the first foil strip (121) facing outwards with respect to the substrate (841);

rolling (842) the elongate shaft (101) of the interventional device (100, 200, 300) across the outwards-facing PSA layer (123) of the first foil strip (121) such that the outwards-facing PSA layer of the first foil strip (123) adheres to the elongate shaft (101) and such that the first foil strip (121) and the ultrasound transducer (102) and the adhesive layer (103) and the second foil strip (122) and the electrical shield layer (105) become attached to the elongate shaft (101) with the adhesive layer (103) facing outwards with respect to the elongate shaft (101);

arranging a protective tube (104) comprising a heat-shrink material over a portion of the elongate shaft (101) to cover at least the ultrasound transducer (102);

applying heat to the protective tube (104) such that the protective tube (104) contracts radially with respect to the longitudinal axis (A - A') of the elogante shaft (101) and such that the adhesive layer (103) adheres to an inner surface of the protective tube (104).

14. Method according to claim 13 wherein the adhesive layer (103) comprises a pressure sensitive adhesive, PSA, layer.

15. Method according to claim 13 wherein the ultrasound transducer transfer stack (840) further comprises at both the first position (D - D') and the second position (E - E') a first electrical conductor (115) and a second electrical conductor (116) disposed between the first foil strip (121) and the second foil strip (122); the first electrical conductor (115) and the second electrical conductor (116) being in electrical contact with the ultrasound transducer (102).

FIG. 1A

FIG. 1B

FIG. 2A

FIG. 2B

FIG. 3A

FIG. 3B

FIG. 4A

FIG. 4B

FIG. 6D

FIG. 6B

FIG. 6C

FIG. 6E

FIG. 5

FIG. 6A

FIG. 7

840

123
115
124
122
105

121
119
116
120

## FIG. 8B

841

123
115
117
102
124
122
105

121
119
118
116
120
103

## FIG. 8C

841

D'

116

E'

102

D

115

E

842

101

A'

A

## FIG. 8A

FIG. 9

FIG. 10

Europäisches
Patentamt

European
Patent Office

Office européen
des brevets

# EUROPEAN SEARCH REPORT

Application Number

EP 18 19 8820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/172544 A1 (ERKAMP RAMON QUIDO [US] ET AL) 22 June 2017 (2017-06-22) | 1,4-9,12 | INV.<br>A61B8/08 |
| Y | * paragraphs [0023], [0031], [0035], [0039], [0049] - [0052]; figures 1-7 *<br>----- | 2,3,10, 11,13-15 | A61M25/01<br>H01L41/047<br>H01L41/313 |
| Y | WO 2018/095793 A1 (KONINKLIJKE PHILIPS NV [NL]) 31 May 2018 (2018-05-31)<br>* page 16 - page 19; figure 5 *<br>----- | 2,3,14 | ADD.<br>A61B17/34 |
| Y,D | WO 2017/013224 A1 (KONINKLIJKE PHILIPS NV [NL]) 26 January 2017 (2017-01-26)<br>* figure 8B *<br>----- | 10,11, 13,15 | |
| A | WO 2017/001525 A1 (KONINKLIJKE PHILIPS NV [NL]; VOLCANO CORP [US])<br>5 January 2017 (2017-01-05)<br>* paragraph [0037] *<br>----- | 1-15 | |

TECHNICAL FIELDS
SEARCHED (IPC)

A61B
H02N
A61M
H01L

The present search report has been drawn up for all claims

| Place of search | Date of completion of the search | Examiner |
|---|---|---|
| Munich | 28 February 2019 | Koprinarov, Ivaylo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 8820

This annex lists the patent family members relating to the patent documents cited in  the above-mentioned European search report.
The members are as contained in the European Patent Office EDP file on
The European Patent Office is in no way liable for these particulars which are merely given for the purpose of information.

28-02-2019

| Patent document cited in search report | | Publication date | Patent family member(s) | | Publication date |
|---|---|---|---|---|---|
| US 2017172544 | A1 | 22-06-2017 | CN | 106163432 A | 23-11-2016 |
| | | | EP | 3131481 A1 | 22-02-2017 |
| | | | JP | 2017512586 A | 25-05-2017 |
| | | | US | 2017172544 A1 | 22-06-2017 |
| | | | WO | 2015155630 A1 | 15-10-2015 |
| WO 2018095793 | A1 | 31-05-2018 | NONE | | |
| WO 2017013224 | A1 | 26-01-2017 | CN | 108028308 A | 11-05-2018 |
| | | | EP | 3326215 A1 | 30-05-2018 |
| | | | JP | 6458199 B2 | 23-01-2019 |
| | | | JP | 2018528604 A | 27-09-2018 |
| | | | RU | 2657114 C1 | 08-06-2018 |
| | | | US | 2018207683 A1 | 26-07-2018 |
| | | | WO | 2017013224 A1 | 26-01-2017 |
| WO 2017001525 | A1 | 05-01-2017 | EP | 3316792 A1 | 09-05-2018 |
| | | | JP | 2018519081 A | 19-07-2018 |
| | | | US | 2018360423 A1 | 20-12-2018 |
| | | | WO | 2017001525 A1 | 05-01-2017 |

EPO FORM P0459

For more details about this annex : see Official Journal of the European Patent Office, No. 12/82

**EP 3 632 334 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016207041 A1

- WO 2017013224 A1

### Non-patent literature cited in the description

- A Non-disruptive Technology for Robust 3D Tool Tracking for Ultrasound-Guided Interventions. **JAY MUNG ; FRANCOIS VIGNON ; AMEET JAIN.** MICCAI 2011, Part I, LNCS 6891. 2011, 153-160